(19) **Europäisches Patentamt / European Patent Office / Office européen des brevets**

(11) **EP 4 732 946 A1**

(12) **EUROPEAN PATENT APPLICATION**
published in accordance with Art. 153(4) EPC

(43) Date of publication:
**29.04.2026  Bulletin 2026/18**

(21) Application number: **24826168.7**

(22) Date of filing: **07.06.2024**

(51) International Patent Classification (IPC):
**B01J 37/04** (2006.01)   **B01J 37/02** (2006.01)
**B01J 37/08** (2006.01)   **B01J 37/00** (2006.01)
**B01J 20/04** (2006.01)   **C07C 51/377** (2006.01)
**C07C 57/04** (2006.01)

(52) Cooperative Patent Classification (CPC):
(C-Sets available)
**B01J 27/1806; B01J 35/40; B01J 35/613;
B01J 37/0009; C07C 51/377**         (Cont.)

(86) International application number:
**PCT/KR2024/007760**

(87) International publication number:
**WO 2024/262846 (26.12.2024 Gazette 2024/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.06.2023  KR 20230081445**

(71) Applicant: **LG Chem, Ltd.**
**Yeongdeungpo-gu
Seoul 07336 (KR)**

(72) Inventors:
• **HAN, Jun Kyu**
  **Daejeon 34122 (KR)**
• **HUANG, Rui**
  **Daejeon 34122 (KR)**
• **LEE, Haeryeong**
  **Daejeon 34122 (KR)**

• **KWON, Heon Yong**
  **Daejeon 34122 (KR)**
• **BANG, Jungup**
  **Daejeon 34122 (KR)**
• **BYUN, Wonbae**
  **Daejeon 34122 (KR)**
• **KIM, Dongeun**
  **Daejeon 34122 (KR)**
• **CHOI, Jae Soon**
  **Daejeon 34122 (KR)**
• **KIM, Mahnjung**
  **Daejeon 34122 (KR)**
• **KIM, Jaeyoung**
  **Daejeon 34122 (KR)**

(74) Representative: **Goddar, Heinz J.**
**Boehmert & Boehmert
Anwaltspartnerschaft mbB
Pettenkoferstrasse 22
80336 München (DE)**

(54) **CATALYST FOR DEHYDRATION OF HYDROXYPROPIONIC ACID AND DERIVATIVE THEREOF AND METHOD FOR PRODUCING SAME**

(57)   The present disclosure relates to a catalyst for dehydration reaction of hydroxypropionic acid and derivatives thereof, and a preparation method thereof. The catalyst according to one embodiment of the present disclosure has excellent lifetime characteristics while having a high reaction yield and selectivity.

EP 4 732 946 A1

(52) Cooperative Patent Classification (CPC): (Cont.)

C-Sets
**C07C 51/377, C07C 57/04**

**Description**

[Technical Field]

The present disclosure relates to a catalyst for dehydration reaction of hydroxypropionic acid and derivatives thereof, and a preparation method thereof.

Cross-reference to Related Application

The present application is based on, and claims priority from, Korean Patent Application No. 10-2023-0081445, filed on June 23, 2023, the disclosure of which is hereby incorporated by reference herein in its entirety.

[Background Art]

Acrylic acid is generally synthesized by a two-step oxidation reaction of propylene, which is a petroleum-derived raw material. However, due to the rapid increase in crude oil prices and concerns about future depletion, research is being conducted to obtain unsaturated carboxylic acids such as acrylic acid, etc. from biomass raw materials.
Mass-production of lactic acid from starch is possible by fermentation, and acrylic acid can be produced through dehydration reaction of lactic acid. In the dehydration reaction of lactic acid, metal phosphate, metal sulfate, or zeolite-based catalysts are mainly used. Among them, metal phosphate is extensively being studied, but it has a problem of a low yield of acrylic acid.
Specifically, International Patent Publication No. WO2011/052178 suggests a synthetic method of synthesizing unsaturated carboxylic acids or derivatives thereof from hydroxycarboxylic acids or derivatives thereof by dehydration reaction using hydroxyapatite ($Ca_{10}(PO_4)_6(OH)_2$) or $Sr_{10}(PO_4)_6(OH)_2$ as a catalyst. However, when acrylic acid is synthesized from lactic acid using hydroxyapatite, the yield of acrylic acid is about 50% to 70%, and when $Sr_{10}(PO_4)_6$ $(OH)_2$, in which Ca in hydroxyapatite is replaced by Sr, is used, the yield of acrylic acid is even lower at about 30%.
Accordingly, there is a need to develop a method and a catalyst, which are able to synthesize unsaturated carboxylic acids such as acrylic acid, etc., or esters thereof in high yields.

[Disclosure]

[Technical Problem]

There is provided a catalyst for dehydration reaction of hydroxypropionic acid and derivatives thereof.
There is also provided a method of preparing the catalyst for dehydration reaction of hydroxypropionic acid and derivatives thereof.

[Technical Solution]

There is provided a catalyst for dehydration reaction of hydroxypropionic acid and derivatives thereof, the catalyst including a molded body in which primary particles of hydroxyapatite are aggregated, wherein a volume average particle size of the primary particles is 15 $\mu$m to 150 $\mu$m; and a P value represented by the following Equation 1 is 3.5 to 19.

$$[\text{Equation 1}]$$

$$P = A*B/C$$

in Equation 1, A represents a volume average particle size ($\mu$m) value of powder, B represents a crush strength (N) value, and C represents a specific surface area ($m^2$/g) value.
According to one embodiment, the particle size of the primary particles may be about 25 $\mu$m to about 100 $\mu$m.
According to one embodiment, a content of alkali metal in the catalyst for dehydration reaction of hydroxypropionic acid and derivatives thereof may be about 3% by weight to about 8% by weight.
According to one embodiment, the alkali metal may be sodium or potassium.
According to one embodiment, an apparent density value of the catalyst for dehydration reaction of hydroxypropionic acid and derivatives thereof may be about 0.8 g/$cm^3$ to about 1.0 g/$cm^3$.
According to one embodiment, a crush strength value of the catalyst for dehydration reaction of hydroxypropionic acid and derivatives thereof may be about 6 N to about 20 N.

According to one embodiment, a specific surface area value of the catalyst for dehydration reaction of hydroxypropionic acid and derivatives thereof may be about 50 m$^2$/g to 90 m$^2$/g.

Meanwhile, there is provided a method of preparing the catalyst for dehydration reaction of hydroxypropionic acid and derivatives thereof, the method including the steps of: preparing a slurry by introducing 1 part by weight to 50 parts by weight of a binder with respect to 100 parts by weight of hydroxyapatite; producing a molded body by molding the slurry; and drying the molded body.

According to one embodiment, the binder may include 0.1 part by weight to 10 parts by weight of isopropyl alcohol with respect to 100 parts by weight of hydroxyapatite.

Meanwhile, there is provided a method of preparing acrylic acid, the method including the step of performing a dehydration reaction of hydroxycarboxylic acid or derivatives thereof in the presence of the above-described catalyst.

As used herein, the terms "the first", "the second", and the like are used to describe a variety of components, and these terms are merely employed to differentiate a certain component from other components.

Further, the terms used in this description are just for explaining exemplary embodiments and it is not intended to restrict the present invention.

The singular expression may include the plural expression unless it is differently expressed contextually.

It must be understood that the term "include", "equip", or "have" in the present description is used only for explaining characteristics taken effect, numbers, steps, components, or combinations thereof, and do not exclude one or more different characteristics, numbers, steps, components, combinations thereof, or the possibility of addition.

Further, in this description, when a layer or an element is mentioned to be formed "on" or "above" layers or elements, it means that each layer or element is directly formed on the layers or elements, or other layers or elements may be additionally formed between the layers, subjects, or substrates..

The present invention may be variously modified and have various forms, and specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

In this description, the hydroxypropionic acid derivatives refer to a group of compounds encompassing hydroxypropionic acid and derivatives thereof, which are carboxylic acids having 3 carbon atoms in the main chain, one of which is replaced by a hydroxy group.

In addition, the dehydration reaction of hydroxypropionic acid and derivatives thereof refers to a reaction in which the hydroxy group and hydrogens bound to the neighboring carbons in the corresponding compound are eliminated, thereby forming a carbon-carbon unsaturated double bond.

Hereinafter, the present invention will be described in detail.

According to one aspect of the present disclosure, provided is a catalyst for dehydration reaction of hydroxypropionic acid and derivatives thereof, the catalyst including a molded body in which primary particles of hydroxyapatite are aggregated, wherein a volume average particle size of the primary particles is 15 $\mu$m to 150 $\mu$m; and a P value represented by the following Equation 1 is 3.5 to 19.

[Equation 1]

$$P = A * B / C$$

in Equation 1, A represents a volume average particle size ($\mu$m) value of powder, B represents a crush strength (N) value, and C represents a specific surface area (m$^2$/g) value.

Apatite compounds refer to compounds having an apatite structure, but usually refer to calcium phosphate series compounds that include calcium and phosphorus and are represented by a chemical formula of $Ca_A(PO_4)_B$-X.

Among them, hydroxyapatite refers to a compound in which X in the chemical formula is a hydroxy group. Such hydroxyapatite compounds may be used as catalysts in the dehydration reaction of hydroxypropionic acid and derivatives thereof for the production of unsaturated carboxylic acids, due to their unique structure and the presence of acid sites.

However, when these calcium phosphate series compounds are used as general hydroxyapatite single-phase catalysts, the yield in the dehydration reaction is not high, in particular, the yield significantly decreases over the reaction time, and their lifetime is very short, and thus there is a problems of low processability.

The present inventors of the present invention have found that, in a method of preparing acrylic acid and the like through the dehydration reaction of hydroxypropionic acid and derivatives thereof using hydroxyapatite as a catalyst, when hydroxyapatite powder is molded and the molded body is used as a catalyst, if the particle size of the hydroxyapatite powder used for molding is controlled within a predetermined range, it is possible to provide a catalyst having very excellent lifetime characteristics while having a high reaction yield and selectivity, thereby completing the present invention.

According to one embodiment of the present disclosure, provided is a catalyst for dehydration reaction of hydroxypropionic acid and derivatives thereof, the catalyst including a molded body in which primary particles of hydroxyapatite are aggregated, wherein a volume average particle size of the primary particles is 15 $\mu$m to 150 $\mu$m; and a P value represented by the following Equation 1 is 3.5 to 19.

[Equation 1]

$$P=A*B/C$$

in Equation 1, A represents a volume average particle size ($\mu$m) value of powder, B represents a crush strength (N) value, and C represents a specific surface area (m$^2$/g) value.

According to one embodiment, the volume average particle size of the primary particles may be 15 $\mu$m to 150 $\mu$m.

In other words, rather than using hydroxyapatite as the catalyst in its crystalline form or in its powder form, the molded body prepared by molding the powder is used as the catalyst. In this regard, the particle size of the powder constituting the molded body, i.e., the primary particles, may be about 15 $\mu$m to about 150 $\mu$m, or about 15 $\mu$m or more, or about 17 $\mu$m or more, or about 20 $\mu$m or more, or about 23 $\mu$m or more, or about 25 $\mu$m or more, and about 150 $\mu$m or less, or about 140 $\mu$m or less, or about 130 $\mu$m or less, or about 120 $\mu$m or less, or about 110 $\mu$m or less, or about 100 $\mu$m or less.

When the size of the primary particles, i.e., hydroxyapatite powder, for producing the molded body catalyst is too small, the density of the powder in the catalyst molded body increases, which tends to increase the physical strength. However, this characteristic may generate a problem that the specific surface area required for the dehydration reaction decreases. When the size of the primary particles, i.e., hydroxyapatite powder, is too large, the strength of the catalyst molded body is weak and thus molding is impossible, and problems of side reactions due to catalyst crush in the dehydration reaction and increased differential pressure in a reactor may occur.

According to one embodiment, the content of alkali metal in the catalyst for dehydration reaction of hydroxypropionic acid and derivatives thereof may be about 3% by weight to about 8% by weight.

In the hydroxyapatite compound which is used as the catalyst for preparing unsaturated carboxylic acid or derivatives thereof, the content of alkali metal in the compound may influence the selectivity in the preparation of unsaturated carboxylic acid and derivatives thereof. Specifically, when the content of alkali metal in the catalyst, particularly, on the surface of the catalyst, is low, the selectivity decreases, but when the content of alkali metal is high, the selectivity may maintain high.

Meanwhile, a molar ratio of calcium to phosphorus (a molar ratio of Ca/P) on the surface of the catalyst affects the yield of the product, i.e., the conversion rate of acrylic acid. When the molar ratio of Ca/P is too high or too low, the yield may decrease.

However, upon preparing unsaturated carboxylic acid or derivatives thereof, the selectivity and the conversion rate exhibit a trade-off relationship, and therefore, it is difficult to improve the selectivity and the conversion rate at the same time.

According to one embodiment of the present disclosure, the content of alkali metal in the catalyst may maintain a high level of alkali metal ratio of about 3% by weight to about 8% by weight, and the content of alkali metal on the surface of catalyst may also maintain at a high level. Accordingly, upon preparing unsaturated carboxylic acid and derivatives thereof, the selectivity and the conversion rate may be further improved by increasing the content of alkali metal on the surface of catalyst, and at the same time, by controlling the mixing ratio with Ca and P.

Specifically, in the catalyst according to one embodiment, the molar ratio of calcium to phosphorus (P) (the molar ratio of Ca/P) on the surface of catalyst may be about 1.0 or more and about 1.2 or less, more specifically, about 1.0 or more, or about 1.05 or more, or about 1.1 or more, and about 1.2 or less, or less than about 1.2, or about 1.15 or less.

Further, a total molar ratio of calcium and alkali metal to phosphorus (a molar ratio of (Ca+M)/P) on the surface of catalyst may be about 1.2 or more, about 1.6 or less, more specifically, about 1.2 or more, or about 1.3 or more, and about 1.6 or less, or less than about 1.6, or about 1.5 or less, or about 1.45 or less, or about 1.4 or less.

The catalyst may have a high content of alkali metal on the surface of the catalyst of about 4% by weight or more and about 8% by weight or less, based on the total weight of elements present on the surface of the catalyst, more specifically, about 4% by weight or more, or about 5% by weight or more, or about 6% by weight or more, or about 6.5% by weight or more, and about 8% by weight or less, or about 7.5% by weight or less, or about 7% by weight or less.

Meanwhile, in the present disclosure, the element contents (at%), based on the atomic weight of elements including phosphorus, calcium, and alkali metal on the surface of catalyst, may be measured through XPS analysis.

From the content of calcium (Ca) (at%), the content of phosphorus (P) element (at%), and the content of alkali metal (M) element (at%) which are calculated through the XPS analysis, the molar ratio of calcium to phosphorus on the surface of catalyst may be obtained from the ratio of Ca element content (Ca at%)/P element content (P at%). In addition, the total molar ratio of calcium and alkali metal to phosphorus may be obtained from the ratio of (Ca at%+ M at%)/(P at%).

Further, the content (% by weight, wt%) of alkali metal present on the surface of catalyst may be calculated from the element content (at%) calculated above.

As described above, upon preparing unsaturated carboxylic acid or derivatives thereof, the selectivity and the conversion rate may be further improved by controlling the content ratio of phosphorus, calcium, and alkali metal on the surface of catalyst and the content of the above elements in the catalyst.

Specifically, the content of alkali metal in the catalyst may be about 3% by weight or more and about 5% by weight or less, based on the total weight of the catalyst. More specifically, the content of alkali metal in the catalyst may be about 3% by weight or more, or about 3.2% by weight or more, and about 5% by weight or less, or about 4.5% by weight or less, about 4% by weight or less, or about 3.7% by weight, based on the total weight of the catalyst.

Further, the molar ratio of calcium to phosphorus (molar ratio of Ca/P) in the catalyst may be about 1.3 or more and about 1.5 or less, more specifically, about 1.3 or more, or about 1.35 or more, or about 1.4 or more, and about 1.5 or less, or about 1.45 or less.

The total molar ratio of calcium and alkali metal (M) to phosphorus (molar ratio of (Ca+M)/P) in the catalyst may be about 1.2 or more and about 2 or less, more specifically, about 1.2 or more, or about 1.5 or more, or about 1.6 or more, or about 1.65 or more, or about 1.7 or more, and about 2 or less, or about 1.9 or less, or about 1.8 or less, or about 1.75 or less.

Meanwhile, in the present disclosure, the content of elements including phosphorus, calcium, and alkali metal in the catalyst may be calculated through inductively coupled plasma mass spectrometry (ICP), more specifically, inductively coupled plasma optical emission spectroscopy (ICP-OES).

According to one embodiment, the alkali metal may be sodium or potassium, and considering the superior improvement effect, sodium may be preferable.

As described above, as the content ratio of phosphorus, calcium, and alkali metal on the catalyst surface and the entire surface is controlled to the optimal range, the catalyst according to the present disclosure may realize high selectivity and high conversion rate at the same time upon preparing unsaturated carboxylic acid or derivatives thereof.

According to one embodiment, the apparent density value of the catalyst for dehydration reaction of hydroxypropionic acid and derivatives thereof may be about 0.8 $g/cm^3$ to about 1.0 $g/cm^3$. When the density is too low, side reactions by catalyst crush during the dehydration reaction may occur due to the weak strength of the catalyst, and there may be a problem that the differential pressure inside the reactor increases. When the density is too high, there may be a problem that the specific surface area decreases.

According to one embodiment, the crush strength value of the catalyst for dehydration reaction of hydroxypropionic acid and derivatives thereof may be about 6 N to about 20 N. In other words, the catalyst according to one embodiment of the present disclosure has a very excellent crush strength value, and thus the catalyst is not easily crushed even when it is packed inside the reactor and exposed to harsh reaction conditions, thereby maintaining the packed state as in the initial state.

According to one embodiment, the specific surface area value of the catalyst for dehydration reaction of hydroxypropionic acid and derivatives thereof may be about 50 $m^2/g$ to about 90 $m^2/g$, or about 50 $m^2/g$ or more, or about 55 $m^2/g$ or more, or about 60 $m^2/g$ or more, and about 90 $m^2/g$ or less, or about 85 $m^2/g$ or less, indicating that it may have a wide specific surface area.

Further, the catalyst for dehydration reaction of hydroxypropionic acid and derivatives thereof may have the P value represented by the following Equation 1 of about 3.5 to about 19, about 3.5 or more, or about 4.0 or more, or about 5.0 or more, or 6.0 or more, about 19 or less, or about 17 or less, or about 15 or less, or about 12 or less, or about 10 or less.

[Equation 1]

$$P = A*B/C$$

in Equation 1, A represents a volume average particle size ($\mu$m) value of powder, B represents a crush strength (N) value, and C represents a specific surface area ($m^2/g$) value.

Equation 1 represents parameterization of factors that may directly influence the strength of the catalyst, which is formed by molding primary particle powder. When the parameter value is too large or too small, the catalyst inactivation time may be shortened, which may cause problems such as deterioration in catalyst performance and deterioration in long-term stability.

Further, according to another aspect of the present disclosure, provided is a method of preparing the catalyst for dehydration reaction of hydroxypropionic acid and derivatives thereof, the method including the steps of: preparing a slurry by introducing 1 part by weight to 50 parts by weight of a binder with respect to 100 parts by weight of hydroxyapatite; producing a molded body by molding the slurry; and drying the molded body.

According to one embodiment, the preparation method may further include the step of grinding hydroxyapatite prior to introducing the binder.

In terms of controlling the particle size of the ground powder to be produced, i.e. the volume average particle size of the primary particles, it may be preferable to use a milling machine, such as a jet mill or pin mill, etc. for grinding.

The binder may include a solvent of water or alcohol series (excluding isopropyl alcohol), and isopropyl alcohol, which is a binder component.

According to one embodiment, the binder may be used in an amount of about 1 part by weight or more, or about 5 parts by weight or more, or about 10 parts by weight or more, and about 70 parts by weight or less, or about 60 parts by weight or less, or about 50 parts by weight or less with respect to 100 parts by weight of hydroxyapatite.

When the amount of use of the binder is too small or too large, there may be problems that the moldability may deteriorate during the preparation of the catalyst, and the strength of the catalyst to be prepared may deteriorate.

However, the content of the binder may vary depending on molding conditions such as the particle size of particles, drying temperature, drying time, etc.

Further, the binder may include about 0.1 part by weight or more, or about 1 part by weight or more, or about 3 parts by weight or more, and about 10 parts by weight or less, or about 9 parts by weight or less, or about 8 parts by weight or less of isopropyl alcohol.

When the content of isopropyl alcohol is too low or too high, there may be problems that the strength of the catalyst to be prepared may deteriorate, or the specific surface area may be reduced.

In the step of preparing the slurry by introducing the binder into hydroxyapatite powder and then mixing the same, the mixing method, etc. are not particularly limited.

Further, when the molded body is produced by molding the slurry, a molding method of using an extruder, etc. may be preferred.

Thereafter, the molded body which is molded in the form of a pellet, etc. is dried. In the drying step, drying may be performed for about 30 minutes to about 24 hours under conditions of about 10°C or higher, or about 20°C or higher, and about 120°C or lower, or about 110°C or lower.

According to one embodiment, the step of calcining the dried catalyst may be further included.

Meanwhile, the present disclosure provides a method of preparing acrylic acid, the method including the step of performing a dehydration reaction of hydroxycarboxylic acid or derivatives thereof in the presence of the above-described catalyst.

Meanwhile, the present disclosure provides a method of preparing unsaturated carboxylic acid and derivatives thereof using the catalyst.

Specifically, the preparation method includes the step of performing a dehydration reaction of hydroxycarboxylic acid or derivatives thereof in the presence of the catalyst for preparing unsaturated carboxylic acid or derivatives thereof.

In the method of preparing unsaturated carboxylic acid and derivatives thereof according to the present disclosure, specific examples of hydroxycarboxylic acid which is a raw material may include lactic acid, citric acid, 3-hydroxypropionic acid, 3-hydroxy-2-methylpropionic acid, 3-hydroxybutanoic acid, 3-hydroxy-2-methylbutanoic acid, 2,3-dimethyl-3-hydroxybutanoic acid, etc., and salts, esters, or dimers thereof, etc.

The hydroxycarboxylic acid or derivatives thereof may be used in the form of an aqueous solution of being dissolved in water, or in the form of a solution of being dissolved in a mixed solvent in which a hydrophilic organic solvent, such as alcohol or ether, etc. is mixed with water.

In this regard, the concentration of the hydroxycarboxylic acid or derivatives thereof is not particularly limited, but may be 20% by weight or more, or 60% by weight or less in consideration of efficiency.

Further, the amount of use of the catalyst in the dehydration reaction may be appropriately selected by considering the type of reactants, the reaction time, etc. For example, hydroxycarboxylic acid or derivatives thereof may be introduced in an amount of 0.05 g or more, 3 g or less per hour, more specifically, 0.1 g or more, 1 g or less per hour, or 0.5 g per hour, based on 1 g of the catalyst

Further, the dehydration reaction may be performed by a continuous reaction using a fixed bed reactor, or by a batch reaction. More specifically, the dehydration reaction may be performed by the continuous reaction, in which the catalyst is charged in the fixed bed reactor, and reactants are allowed to react by continuously introducing the same into the reactor, thereby continuously preparing the product.

During the continuous reaction using the fixed bed reactor, an inert gas such as nitrogen, argon, or helium, etc. may be used as a carrier gas. The input amount of the carrier gas is not particularly limited, and may be appropriately determined according to reaction conditions such as the input amount of the reactants, etc. For example, the carrier gas may be introduced in an amount of 5 ml/min or more or 500 ml/min or less per 1 g of the catalyst.

Further, the dehydration reaction may be performed at a temperature of 300°C or higher, 500°C or lower, more specifically, at a temperature of 300°C or higher, or 350°C or higher, or 360°C or higher, and 500°C or lower, or 450°C or lower, or 380°C or lower.

Further, the dehydration reaction may be performed at a pressure of 0.5 bar or more, 5 bar or less, more specifically, at 0.5 bar or more, or 0.8 bar or more, and 5 bar or less, or 2 bar or less, and much more specifically, under normal pressure ($1 \pm 0.2$ bar) condition.

Further, the reactant hydroxycarboxylic acid or derivative thereof may be introduced at a weight hourly space velocity (WHSV) of 0.05 h$^{-1}$ or more, 3 h$^{-1}$ or less, more specifically, at a WHSV of 0.05 h$^{-1}$ or more, or 0.1 h$^{-1}$ or more, or 0.3 h$^{-1}$ or more, and 3 h$^{-1}$ or less, or 1 h$^{-1}$ or less or 0.8 h$^{-1}$ or less.

When the reaction temperature is higher than 500°C, or the reaction pressure is lower than 0.5 bar, or the reactant supply rate WHSV is lower than 0.1 h$^{-1}$, the catalyst activity may excessively increase, and thus there is concern about progression of hydrocracking side reactions, resulting in a decrease in the selectivity. On the contrary, when the reaction temperature is lower than 300°C, the reaction pressure is higher than 5 bar, or the reactant supply rate WHSV is higher than 1 h$^{-1}$, the conversion rate may decrease, and thus other reaction conditions may have to be harshly increased, resulting in a shortened catalyst lifetime and increased costs in the step of separating and recovering the product.

By the dehydration reaction as described above, at least part of the hydroxycarboxylic acid or derivatives thereof is converted into unsaturated carboxylic acid or derivatives thereof.

The method of preparing unsaturated carboxylic acid or derivatives thereof according to the present disclosure improves the conversion rate of unsaturated carboxylic acid by using the above-described catalyst, thereby preparing unsaturated carboxylic acid and derivatives thereof in high yields.

[Effect of the Invention]

A catalyst according to one embodiment of the present disclosure has very excellent lifetime characteristics while having a high reaction yield and selectivity.

[Detailed Description of the Embodiments]

Hereinafter, the actions and effects of the present invention will be described in more detail with reference to the specific embodiments of the present invention. However, these embodiments are provided only for illustrating the present invention, but the scope of the present invention is not defined thereby.

<Example>

**Preparation of Catalyst**

**Example 1**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 26 $\mu$m.

With respect to 100 parts by weight of the obtained particles, 28 parts by weight of water and 5 parts by weight of isopropyl alcohol were added as a binder, followed by mixing well, and then pellets of about 2.5 mm in size were prepared using an extruder. The pellets were dried at about 60°C for about 12 hours to obtain a catalyst molded body in the form of a solid pellet.

**Example 2**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 42 $\mu$m.

With respect to 100 parts by weight of the obtained particles, 28 parts by weight of water and 5 parts by weight of isopropyl alcohol were added as a binder, followed by mixing well, and then pellets of about 2.5 mm in size were prepared using an extruder. The pellets were dried at about 60°C for about 12 hours to obtain a catalyst molded body in the form of a solid pellet.

**Example 3**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 60 $\mu$m.

With respect to 100 parts by weight of the obtained particles, 25 parts by weight of water and 5 parts by weight of isopropyl alcohol were added as a binder, followed by mixing well, and then pellets of about 2.5 mm in size were prepared using an extruder. The pellets were dried at about 30°C for about 12 hours to obtain a catalyst molded body in the form of a solid pellet.

**Example 4**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 80 $\mu$m.

With respect to 100 parts by weight of the obtained particles, 35 parts by weight of water and 5 parts by weight of isopropyl alcohol were added as a binder, followed by mixing well, and then pellets of about 2.5 mm in size were prepared using an extruder. The pellets were dried at about 100°C for about 12 hours to obtain a catalyst molded body in the form of a solid pellet.

**Example 5**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 90 $\mu$m.

With respect to 100 parts by weight of the obtained particles, 28 parts by weight of water and 5 parts by weight of isopropyl alcohol were added as a binder, followed by mixing well, and then pellets of about 2.5 mm in size were prepared using an extruder. The pellets were dried at about 70°C for about 12 hours to obtain a catalyst molded body in the form of a solid pellet.

**Comparative Example 1**

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 12 $\mu$m.

With respect to 100 parts by weight of the obtained particles, 28 parts by weight of water and 5 parts by weight of isopropyl alcohol were added as a binder, followed by mixing well, and then pellets of about 2.5 mm in size were prepared using an extruder. The pellets were dried at about 120°C for about 12 hours to obtain a catalyst molded body in the form of a solid pellet.

## Comparative Example 2

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 8 $\mu$m.

With respect to 100 parts by weight of the obtained particles, 28 parts by weight of water and 5 parts by weight of isopropyl alcohol were added as a binder, followed by mixing well, and then pellets of about 2.5 mm in size were prepared using an extruder. The pellets were dried at about 60°C for about 12 hours to obtain a catalyst molded body in the form of a solid pellet.

## Comparative Example 3

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 160 $\mu$m.

With respect to 100 parts by weight of the obtained particles, 28 parts by weight of water and 10 parts by weight of isopropyl alcohol were added as a binder, followed by mixing well, and then pellets of about 2.5 mm in size were prepared using an extruder. The pellets were dried at about 60°C for about 12 hours to obtain a catalyst molded body in the form of a solid pellet.

## Comparative Example 4

35 g of HAP and 65 g of CaPP were mixed and placed in a high-temperature and highpressure reactor, and then 500 ml of 1 M NaOH solution was introduced and reacted for 4 hours under conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 220 $\mu$m.

With respect to 100 parts by weight of the obtained particles, 35 parts by weight of water was added as a binder, followed by mixing well, and then pellets of about 2.5 mm in size were prepared using an extruder. The pellets were dried at about 150°C for about 12 hours to obtain a catalyst molded body in the form of a solid pellet.

## Comparative Example 5

35 g of HAP and 65 g of CaPP were mixed and placed in a high-temperature and highpressure reactor, and then 500 ml of 1 M NaOH solution was introduced and reacted for 4 hours under the conditions of 150°C and 5 atm. The resulting white precipitate was filtered and washed to obtain a powdered Na-HAP component catalyst (yield: 90%).

The powdered catalyst thus obtained was finely ground using a jet mill or pin mill. During the grinding, the particle size of the prepared particles was controlled by varying the conditions of the milling machine.

The particle size was measured using a wet particle size distribution (PSD) analyzer (Microtrac S3500), and as a result, it was measured that the volume average particle size was about 500 $\mu$m.

With respect to 100 parts by weight of the obtained particles, 25 parts by weight of water and 8 parts by weight of isopropyl alcohol were added as a binder, followed by mixing well, and then pellets of about 2.5 mm in size were prepared, but the molding was failed.

## Measurement of Crush strength

The crush strength of single pellets was measured using a strength tester according to ASTM D4179.

**Measurement of Density**

Tap density was measured with the same number of taps using a COPLEY Tap density tester.

**Measurement of Specific Surface Area**

The specific surface area value of the catalyst was calculated from the amount of nitrogen gas adsorption under liquid nitrogen temperature (77 K) using BELSORP-mino II of BEL Japan.

**Progression of Lactic Acid Dehydration Reaction**

A fixed bed reactor was charged with about 0.71 g of the catalyst prepared in Preparation Example 1, and dehydration reaction was performed by supplying a 30wt% aqueous solution of lactic acid at a WHSV of 0.67 $h^{-1}$ under conditions of a reaction temperature of 370°C and normal pressure ($1\pm0.2$ bar).

The reaction product was received and removed during the initial 4 hours of the reaction which is a reaction stabilization time, and then the liquid product obtained for 2 hours was recovered as a liquid sample using a 4°C cooling collector while continuously performing the reaction.

The recovery rate of acrylic acid was monitored, and the point at which the recovery rate showed a maximum value was confirmed, indicating that the reaction was activated. Then, based on the time when 24 hours passed, the time when the recovery rate decreased by 10% from the reference value was determined as the catalyst inactivation time.

The measurement results are summarized in the table below.

[Table 1]

|  | Bulk density (g/ml) | Crush strength (N) | Specific surface area (m$^2$/g) | (*)P=A*B/C | Inactivation time (hour) |
|---|---|---|---|---|---|
| Example 1 | 0.91 | 15.8 | 64.3 | 6.4 | 48 |
| Example 2 | 0.92 | 15.9 | 79.1 | 8.4 | 50 |
| Example 3 | 0.96 | 10.1 | 80.5 | 7.5 | 55 |
| Example 4 | 0.91 | 9.2 | 82.4 | 8.9 | 58 |
| Example 5 | 0.91 | 9.0 | 83.5 | 9.7 | 59 |
| Comparative Example 1 | 0.93 | 15.5 | 58.4 | 3.2 | 38 |
| Comparative Example 2 | 0.93 | 15.7 | 45.5 | 2.8 | 36 |
| Comparative Example 3 | 0.85 | 8.4 | 70.4 | 19.1 | 41 |
| Comparative Example 4 | 0.81 | 5.9 | 65.2 | 19.9 | 43 |
| (*)A represents the particle size, B represents the crush strength, and C represents the specific surface area value. | | | | | |

Referring to the table above, some of Comparative Examples showed that the inactivation time reached a certain level. However, in this case, the strength was significantly reduced. When the crush strength of the catalyst was low, as in these Comparative Examples, the catalyst molded body located under the catalyst packing layer was crushed, which received a high load as the reaction progressed, and eventually, the catalyst packing layer fails to maintain its shape, thereby damaging the long-term stability of the reaction.

In contrast, it was confirmed that the catalysts according to Examples of the present invention had the wide specific surface area, and at the same time, very high crush strength, and its long-term stability was very excellent, which may be inferred by the inactivation time.

**Claims**

1. A catalyst for dehydration reaction of hydroxypropionic acid and derivatives thereof, the catalyst comprising a molded body in which primary particles of hydroxyapatite are aggregated, wherein a volume average particle size of the primary particles is 15 $\mu$m to 150 $\mu$m; and a P value represented by the following Equation 1 is 3.5 to 19:

[Equation 1]

$$P=A*B/C$$

wherein in Equation 1, A represents the volume average particle size ($\mu$m) value of powder, B represents a crush strength (N) value, and C represents a specific surface area ($m^2$/g) value.

2. The catalyst for dehydration reaction of hydroxypropionic acid and derivatives thereof of claim 1, wherein the volume average particle size of the primary particles is 25 $\mu$m to 100 $\mu$m.

3. The catalyst for dehydration reaction of hydroxypropionic acid and derivatives thereof of claim 1, wherein a content of alkali metal is 3% by weight to 8% by weight.

4. The catalyst for dehydration reaction of hydroxypropionic acid and derivatives thereof of claim 3, wherein the alkali metal is sodium or potassium.

5. The catalyst for dehydration reaction of hydroxypropionic acid and derivatives thereof of claim 1, wherein an apparent density value is 0.8 $g/cm^3$ to 1.0 $g/cm^3$.

6. The catalyst for dehydration reaction of hydroxypropionic acid and derivatives thereof of claim 1, wherein a crush strength value is 6 N to 20 N.

7. The catalyst for dehydration reaction of hydroxypropionic acid and derivatives thereof of claim 1, wherein a specific surface area value is 50 $m^2$/g to 90 $m^2$/g.

8. A method of preparing a catalyst for dehydration reaction of hydroxypropionic acid and derivatives thereof, the method comprising the steps of:

preparing a slurry by introducing 1 part by weight to 50 parts by weight of a binder with respect to 100 parts by weight of hydroxyapatite;
producing a molded body by molding the slurry; and
drying the molded body.

9. The method of claim 8, wherein the binder includes 0.1 part by weight to 10 parts by weight of isopropyl alcohol with respect to 100 parts by weight of hydroxyapatite.

10. A method of preparing acrylic acid, the method comprising the step of performing a dehydration reaction of hydroxycarboxylic acid or derivatives thereof in the presence of the catalyst of claim 1.

## INTERNATIONAL SEARCH REPORT

| International application No. |
|---|
| **PCT/KR2024/007760** |

| **A.** | **CLASSIFICATION OF SUBJECT MATTER** |
|---|---|

**B01J 37/04**(2006.01)i; **B01J 37/02**(2006.01)i; **B01J 37/08**(2006.01)i; **B01J 37/00**(2006.01)i; **B01J 20/04**(2006.01)i; **C07C 51/377**(2006.01)i; **C07C 57/04**(2006.01)i

According to International Patent Classification (IPC) or to both national classification and IPC

| **B.** | **FIELDS SEARCHED** |
|---|---|

Minimum documentation searched (classification system followed by classification symbols)

B01J 37/04(2006.01); B01J 21/10(2006.01); B01J 23/745(2006.01); B01J 27/18(2006.01); B01J 27/20(2006.01); B01J 37/00(2006.01); B01J 37/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히드록시아파타이트(hydroxyapatite), 촉매(catalyst), 히드록시프로피온산 (hydroxypropionic acid), 바인더(binder)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | KR 10-2012-0025888 A (KOREA RESEARCH INSTITUTE OF CHEMICAL TECHNOLOGY) 16 March 2012 (2012-03-16)<br>See claims 1-9; paragraphs [0008]-[0030]; and table 1. | 1-7,10 |
| Y | | 8-9 |
| Y | KR 10-2017-0103532 A (LG CHEM, LTD.) 13 September 2017 (2017-09-13)<br>See claims 1-9; and paragraphs [0047]-[0050]. | 8-9 |
| A | KR 10-2022-0074721 A (LG CHEM, LTD.) 03 June 2022 (2022-06-03)<br>See claims 1-2. | 1-10 |
| A | KR 10-2022-0052742 A (KOREA RESEARCH INSTITUTE OF CHEMICAL TECHNOLOGY) 28 April 2022 (2022-04-28)<br>See claim 1. | 1-10 |

✓ Further documents are listed in the continuation of Box C.   ✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 September 2024** | **11 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/007760** |

**C.    DOCUMENTS CONSIDERED TO BE RELEVANT**

| Category* | Citation of document, with indication, where appropriate, of the relevant passages | Relevant to claim No. |
|---|---|---|
| A | US 4729978 A (SAWICKI, R. A.) 08 March 1988 (1988-03-08)<br>See abstract; and claim 1. | 1-10 |

Form PCT/ISA/210 (second sheet) (July 2022)

## INTERNATIONAL SEARCH REPORT
### Information on patent family members

International application No.

**PCT/KR2024/007760**

| Patent document cited in search report | | | Publication date (day/month/year) | Patent family member(s) | | | Publication date (day/month/year) |
|---|---|---|---|---|---|---|---|
| KR | 10-2012-0025888 | A | 16 March 2012 | KR | 10-1187804 | B1 | 09 October 2012 |
| KR | 10-2017-0103532 | A | 13 September 2017 | CN | 107847916 | A | 27 March 2018 |
| | | | | EP | 3308854 | A1 | 18 April 2018 |
| | | | | JP | 2018-518360 | A | 12 July 2018 |
| | | | | JP | 6569118 | B2 | 04 September 2019 |
| | | | | KR | 10-2001144 | B1 | 17 July 2019 |
| | | | | US | 10518250 | B2 | 31 December 2019 |
| | | | | US | 2018-0214854 | A1 | 02 August 2018 |
| | | | | WO | 2017-150830 | A1 | 08 September 2017 |
| KR | 10-2022-0074721 | A | 03 June 2022 | CN | 115427145 | A | 02 December 2022 |
| | | | | EP | 4108327 | A1 | 28 December 2022 |
| | | | | JP | 2023-520547 | A | 17 May 2023 |
| | | | | JP | 7376054 | B2 | 08 November 2023 |
| | | | | US | 2023-0159427 | A1 | 25 May 2023 |
| | | | | WO | 2022-114519 | A1 | 02 June 2022 |
| KR | 10-2022-0052742 | A | 28 April 2022 | KR | 10-2448165 | B1 | 28 September 2022 |
| US | 4729978 | A | 08 March 1988 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230081445

- WO 2011052178 A